# EUROPEAN PATENT SPECIFICATION

(11) **EP 1 340 437 B1**
(45) Date of publication and mention of the grant of the patent: **16.01.2008**
(21) Application number: 01955592.9
(22) Date of filing: 08.08.2001
(51) Int. Cl.: A45D 44/22, B63C 11/00

(54) **BEAUTY CULTURE DEVICE**
SCHÖNHEITSPFLEGEVORRICHTUNG
DISPOSITIF DE SOINS DE BEAUTE

(30) Priority: 28.11.2000 JP 2000360479
(43) Date of publication of application: 03.09.2003
(73) Proprietor: Yaman Ltd., Tokyo 135-0045 (JP)
(72) Inventor: Yamazaki, Iwao, c/o Yaman Ltd, Tokyo 135-0045 (JP); Izawa, Yoshihiro, c/o Yaman Ltd, Tokyo 135-0045 (JP)
(74) Representative: Newstead, Michael John
(86) International application number: PCT/JP2001/006818
(87) International publication number: WO 2002/043522

(56) References cited:
- JP-A- 1 317 406
- JP-A- 60 021 758
- JP-A- 61 203 906
- JP-U- 61 096 821
- JP-U- 62 007 919
- JP-Y2- 52 007 323
- JP-Y2- 56 037 721

## Description

### Technical Field:

The present invention relates to an apparatus for giving women beauty treatments for the face by making electric pulse current of low frequency flow in the face, thereby making the face spruce or tidy.

### Background Art:

Small, spruce faces give a pleasing impression to people. The causes for enlarging the face are: sagging cheeks of the face, increasingly conspicuous with age; swelling due to mental stresses; and fatty cheeks and double chins increasingly conspicuous when women are obese. This can be effectively treated by removing extra amount of fat from the cheeks and chins. Also, the sagging and swelling can be reduced more or less by the exercising of the face.

To remove extra amount of fat from the face or reduce the sagging and swelling in the face it is necessary to effectively move the *facial emotion-expression* muscles everyday by opening the mouth largely, pressing the lips together and such like. Inconveniently such unusual facial exercise cannot be performed in presence of people.

It is well known that by making pulse current of low-frequency flow in living bodies for stimulation cells can be effectively activated to cause muscular contraction on impulsion. Likewise, application of impulsive stimulation on the face causes removal of extra fat from the face by burning it as would be caused by actual exercise. And the sagging can be removed from the cheeks, and wrinkles can be removed from the face, too. Also, the flow of blood and lymph can be expedited to effectively reduce the swelling in the face.

One object of the present invention is to provide an apparatus for giving women beauty treatments for the face by making electric pulse current of low frequency flow in the face, thereby making the *facial motion-expression* muscles move naturally without recourse to actual exercise.

### Disclosure of Invention:

To attain this object an apparatus for giving women beauty treatments for the face according to the present invention comprises a mask lined with an air bag of water-permeable polymer, which has flat electrodes of soft material attached to the areas of the mask to be applied to the opposite cheeks; a low-frequency pulse source connected to the flat electrodes of the mask; and a compressor and a humidifier both connected to the air bag to supply the air bag with steam for inflation and deflation, as defined in claim 1.

It may further comprise a vibrator attached to the mask for vibration, as described in claim 2.

It may further comprise a heater connected to the air bag for supplying the bag with warm air, as described in claim 3,

It may further comprise a cooler connected to the air bag for supplying the bag with cold air, as defined in claim 4.

### Brief Description of Drawings:

Fig.1 is a front view of a facial beauty treatment apparatus according to the present invention;
Fig.2 is a piping diagram showing how a compressor is connected to the facial beauty treatment apparatus;
Fig.3 is a piping diagram showing a modification of a selected part of Fig.2;
Fig.4 shows how the air pressure varies with time for effective removal of the sagging from the face;
Fig.5 shows how the air pressure varies with time for effective removal of the fat from the face;
Fig.6 shows how the air pressure varies with time for making the face skin smooth;
Fig.7 shows a wiring diagram of a pulse source;
Fig.8 illustrates the effect caused on the depth under the face in the toning mode;
Fig.9 illustrates the effect caused on the depth under the face in the drainage mode; and
Fig. 10 illustrates the effect caused on the depth under the face in the composite mode.

### Best Mode for Carrying Out the Invention:

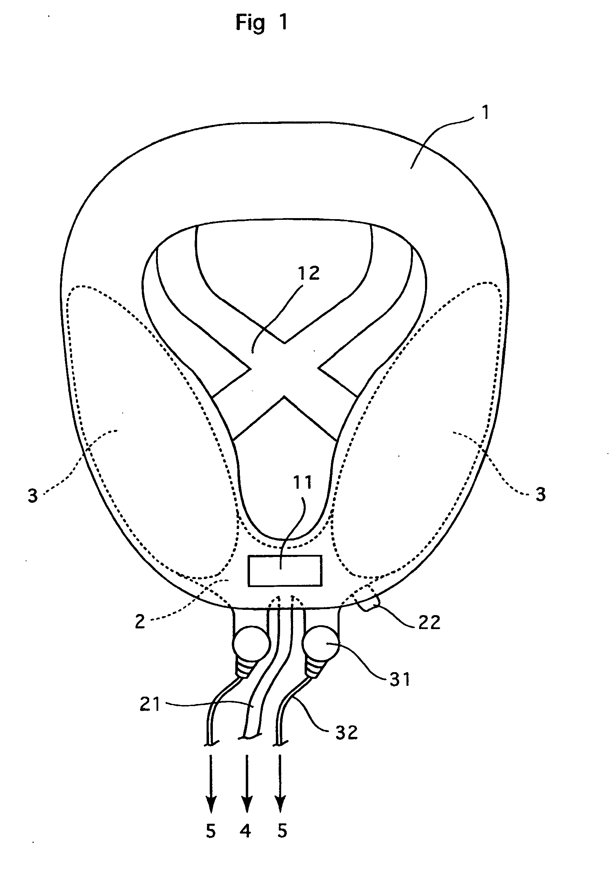

Referring to these figures, particularly to Fig.1, a facial beauty treatment apparatus according to one preferred embodiment of the present invention is described below.

Referring to Fig.1, the facial beauty treatment apparatus comprises a mask 1 covering the part of the face other than the eyes and the nose. The mask 1 is a "V"-shaped air bag 2, which has two flat electrodes 3 attached to the opposite side areas of the air bag 2 to be applied to the opposite cheeks and chin of the face.

The mask 1 has a vibrator 11 on its front side in the vicinity of the part to be applied to the chin.

The vibrator 11 is an electric motor whose rotor has an off-center weight fixed thereto.

The mask 1 has band fasteners 12 attached to its rear side for fastening the mask 1 to the face.

The air bag 2 has a tube 21 connected to its lower side, and an air compressor 4 is connected to the air bag 2, which is equipped with a release valve 22.

The flat electrodes 3 are connected to a low-frequency pulse source 5 by connecting electric cords 32 to the snap fasteners 31 of the electrodes 3.

The flat electrodes 3 are composed of pieces of carbon-coated urethane, which are sewn on or welded to the rear side of the air bag 2 with its coating side exposed.

Otherwise, liquid carbon is applied onto the rear side of the air bag 2 to form a multi-layer carbon printing on the air bag 2.

The flat electrodes 3 are attached or formed to be flush with the rear surface of the air bag 2, preventing any feeling of existence of foreign substance or the peeling-off from the air bag 2.

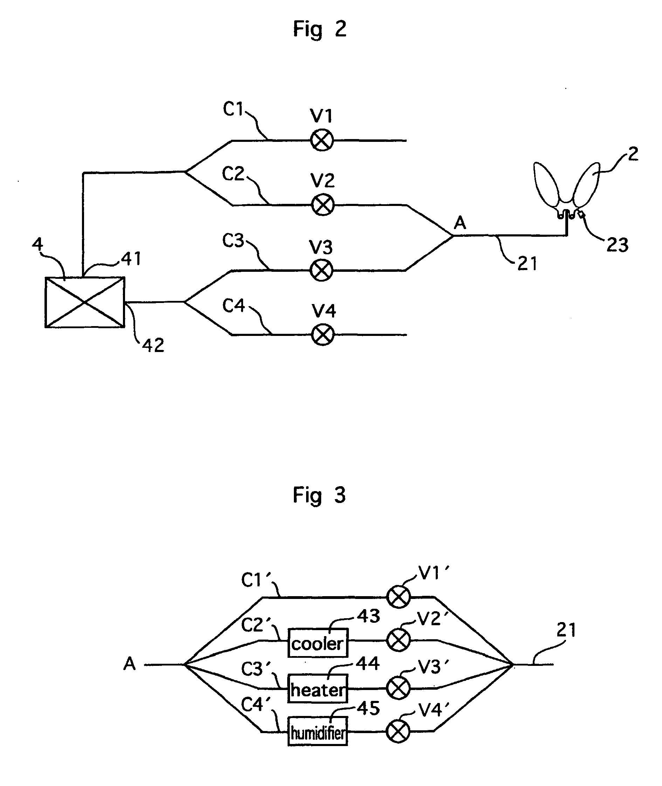

Referring to Fig.2, the compressor 4 is connected to branch pipes C1 and C2 via its inspiratory port 41 and branch pipes C3 and C4 via its expiratory port 42. The branch pipes C2 and C3 are connected to a common point A via associated valves V2 and V3, and a tube 21 connects the common point A to the air bag 2.

The branch pipes C1 and C4 with valves V1 and V4 are open at their ends.

The air bag 2 is equipped with a pressure sensor 23.

The piping arrangement is constructed as described above, and is used to periodically vary the air pressure of the air bag for inflation and contraction as follows: first, the valve V1 of the branch pipe C1 is opened; the valve V2 of the branch pipe C2 is closed; the valve V3 of the branch pipe C1 is opened; and the valve V4 of the branch pipe C4 is closed.

Then, the air bag 2 is inflated with air.

When the pressure sensor 23 detects the air pressure reaching the uppermost, the valve V1 of the branch pipe C1 is closed; the valve V2 of the branch pipe C2 is opened; the valve V3 of the branch pipe C1 is closed; and the valve V4 of the branch pipe C4 is opened.

Then, the air pressure in the airbag 2 is lowered, deflating the air bag 2.

When the pressure sensor 23 detects the air pressure reaching the lowermost, the air bag 2 is inflated with air to increase its inner pressure. The valve control as described is repeated.

When it is desired that the air bag 2 is supplied with cold air, warm air or wet and hot air other than air, the piping is modified as shown in Fig.3.

Specifically branch pipes C1', C2', C3', and C4' diverge from the point "A" to be ended with valves V1', V2', V3' and V4', which are connected to the pipe 21.

A cooler 43 is connected upstream side of the valve V2' in the branch pipe C2'; a heater 44 is connected upstream side of the valve V3' in the branch pipe C3'; and a humidifier 45 is connected upstream side of the valves V4' in the branch pipe C4'.

When the air bag 2 is inflated with air, the valves V2', V3' and V4' are closed, and the valve V1' is opened.

When the air bag 2 is inflated with cold air, the valves V1', V3' and V4' are closed, and the valve V2' is opened to allow air to pass through the cooler 43.

When the air bag 2 is inflated with warm air, the valves V1', V2' and V4' are closed, and the valve V3' is opened to allow air to pass through the heater 43.

When the air bag 2 is inflated with wet air, the valves V1', V2' and V3' are closed, and the valve V4' is opened to allow air to pass through the humidifier 43.

When the air bag 2 is inflated with cold air, the capillary vessels of the face are made to contract for a moment, and warm blood flushes into the capillary vessels to raise the temperature of the face for preventing the face from being too cold.

Alternate supply of cold and warm air to the air bag 2 will expedite the blood circulation in the face.

The air bag 2 is made of water-permeable polymer, and it is inflated with steam to make pores in the face open wide, thereby removing grease or fat from the pores.

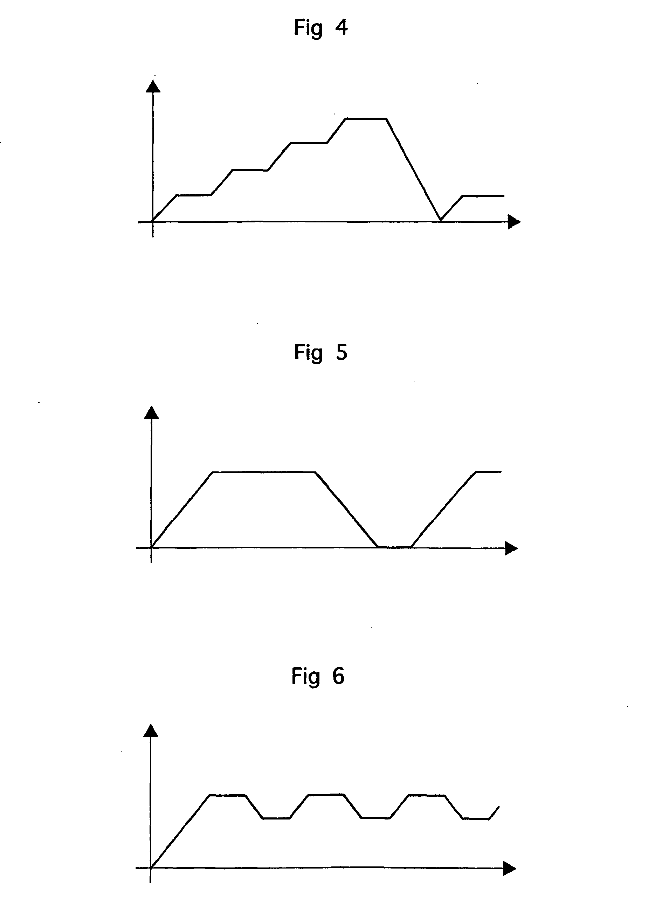

Figs.4, 5 and 6 are graphic representations showing how the air pressure is controlled to cause the massaging effect by inflating and deflating the air bag 2.

Specifically Fig.4 shows a graphic pattern of air pressure effective for strengthening the face muscles and removing the sags from the chin and cheeks.

Fig.5 shows a graphic pattern of air pressure effective for tightening the face muscles and removing the fat from the face.

Fig.6 shows a graphic pattern of air pressure effective for deeply stimulating the face skin and making the skin smooth.

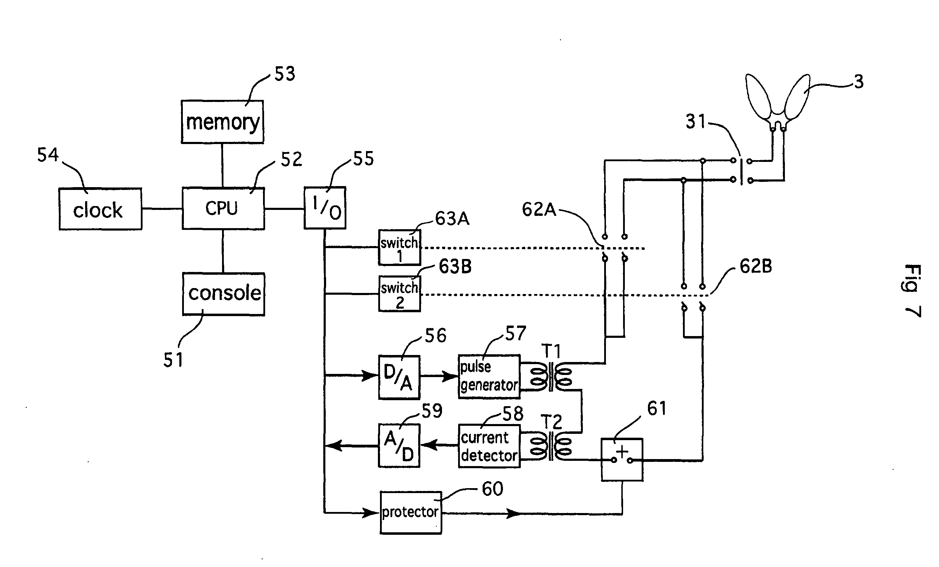

Fig.7 is a block diagram of a pulse supply 5 used in the facial beauty treatment apparatus according to the present invention.

In the drawing a CPU 52 has a console 51, an associated memory 53, a clock pulse generator 54 and an interface 55 connected thereto. The CPU 52 is connected via the interface 55, to: switching units 63A and 63B, which actuate associated switches 62A and 62B; a digital-to-analogue converter 56; an analogue-to-digital converter 59; and a protector-and-breaker 60 and 61. The digital-to-analogue converter 56 is connected to a pulse generator 57, which is connected to a transformer T1. The analogue-to-digital converter 59 is connected to a current detector 58, which is connected to a transformer T2. The secondary windings of the transformers T1 and T2 are series-connected, and the series-connection of the secondary windings is connected to the flat electrodes 3 of the mask 1 via the breaker 61 and switches 62A and 62B. In operation a user selects the kind of treatment and feeding pattern on the console 51, thereby making the CPU 52 retrieve required pieces of information from the memory 53, and then a train of clock pulses from the clock pulse generator 54 are divided in frequency, depending on the selected kind of treatment and feeding pattern, thus, providing required digital triggering signals, which are directed to the pulse generator 57 via the interface I/O 55 and D/A converter 56 to provide a train of pulses, which are modified in terms of width and frequency. The primary winding of the transformer T1 is supplied with the train of pulses thus modified.

The current detector 58 is connected to the transformer T2 on its primary side to watch the current in respect of whether the current increases beyond a threshold.

The current thus detected is directed to the CPU 52 via the A/D converter 59 and I/O interface 55. When the current increases beyond a threshold, the protector 60 actuates the circuit breaker 61

The switches 62A and 62B are connected to the opposite terminals of the series-connection of the secondary windings of the transformers T1 and T2, and the switches 62A and 62B are connected to the flat electrodes 3 via associated conductor snap fasteners 31.

The switches 62A and 62B are photo-coupler type of switching circuits, responsive to signals from the switching units 63A and 63B for turning on their associated photo-couplers.

Thus, the flat electrodes 3 are supplied with pulse current according to the selected feeding pattern for attaining the desired facial beauty treatment.

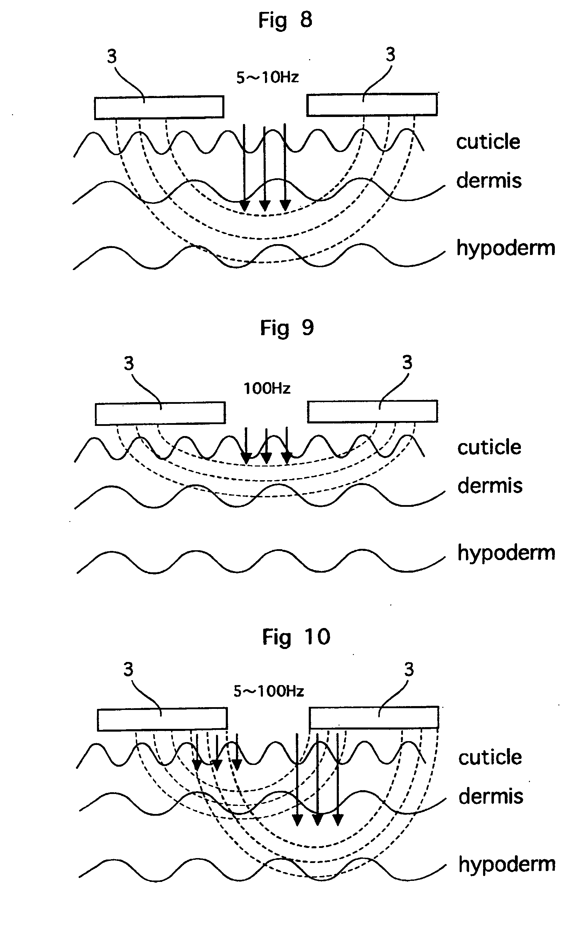

Referring to Fig.8, application of pulses of relatively low frequency (5 to 10 Hz) to the face in toning mode causes stimulation of the face skin from the cuticle deep to the hypoderm, thereby making the muscles of the face move for removing the sags from the chin and the cheeks. Referring to Fig.9, application of pulses of relatively high frequency (20 to 100 Hz) to the face in drainage mode causes stimulation of the face skin from cuticle deep to the dermis, thereby expediting the circulation of the blood and lymph in the face for removing the swelling from the face. Finally referring to Fig.10, application of pulses of wide frequency range (5 to 100 Hz) to the face in the composite mode causes stimulation of the face skin from the cuticle deep to the hypoderm, thereby making the face skin resilient and fresh-looking.

In effecting the facial beauty treatment the air bag 2 is applied to the inner surface of the mask 1, and the mask 1 is applied to the face to be fastened to the face by winding and tightening the band fasteners around the head.

The kind of treatment to be effected is indicated, and then the start button is depressed on the console 51, thus supplying current pulses of selected frequency and duration to the flat electrodes 3.

Simultaneously the vibrator 11 is put in operation to vibrate the mask 1 for stimulating the face with a small amplitude.

When the compressor 4 is put in operation, the air bag 2 is inflated and deflated alternately to strongly massage the face.

When the cooler 43 is put in operation, the air bag 2 is filled with cold air to descend the temperature of the face skin.

When the heater 44 is put in operation, the air bag 2 is filled with warm air to expedite the circulation of blood in the face.

When the humidifier 45 is put in operation, the air bag 2 is filled with wet and warm air or steam to open the pores wide.

### Industrial Applicability:

As described above, an apparatus for giving women beauty treatments for the face according to the present invention uses a mask lined with an air bag of water-permeable polymer, which has flat electrodes of soft material attached to the areas of the mask to be applied to the opposite cheeks, a low-frequency pulse source connected to the flat electrodes of the mask and a compressor and a humidifier both connected to the air bag to supply the air bag with steam for inflation and deflation.

The flat electrodes can be closely applied to the cheeks when the air bag is inflated with air, thus allowing current pulses of low frequency to effectively flow in the cheeks, thereby making the usually motion-less muscles in the face move lively. Thus, the face is significantly reduced in appearance.

The muscles of the part of the face which produces the emotional expression on the face can be stimulated to make the face naturally attractive. The pores of the face can be opened wide to remove the fat and clean inside.

## Claims

1. An apparatus for giving women beauty treatments for the face comprising a mask (1) lined with an air bag (2) of water-permeable polymer, which has flat electrodes (3) of soft material attached to the areas of the mask (1) to be applied to the opposite cheeks; a low-frequency pulse source (5) connected to the flat electrodes (3) of the mask (1); and a compressor (4) and a humidifier (45) both connected to the air bag (2) to supply the air bag (2) with steam for inflation and deflation.

2. An apparatus for giving women beauty treatments for the face according to claim 1 wherein it further comprises a vibrator (11) attached to the mask (1) for vibration.

3. An apparatus for giving women beauty treatments for the face according to claim 1 wherein it further comprises a heater (44) connected to the air bag (2) for supplying the bag (2) with warm air.

4. An apparatus for giving women beauty treatments for the face according to claim 1 wherein it further comprises a cooler (43) connected to the air bag (2) for supplying the bag with cold air.

## Patentansprüche

1. Vorrichtung zur Durchführung von Schönheitsbehandlungen für das Gesicht von Frauen, die Folgendes umfasst: eine Maske (1), die mit einem Luftkissen (2) aus wasserdurchlässigem Polymer ausgekleidet ist, die an den Bereichen der Maske (1) befestigte Flachelektroden (3) aus einem weichen Material aufweist, die auf die gegenüberliegenden Wangen aufzulegen sind; eine niederfrequente Impulsquelle (5), die mit den Flachelektroden (3) der Maske (1) verbunden ist; und einen Kompressor (4) und einen Befeuchter (45), die beide mit dem Luftkissen (2) verbunden sind, um das Luftkissen (2) zum Aufblasen und Ablassen mit Dampf zu versorgen.

2. Vorrichtung zur Durchführung von Schönheitsbehandlungen für das Gesicht von Frauen nach Anspruch 1, die weiter einen an der Maske (1) befestigten Vibrator (11) zum Vibrieren umfasst.

3. Vorrichtung zur Durchführung von Schönheitsbehandlungen für das Gesicht von Frauen nach Anspruch 1, die weiter eine Heizvorrichtung (44) umfasst, die mit dem Luftkissen (2) zur Versorgung des Kissens (2) mit Warmluft verbunden ist.

4. Vorrichtung zur Durchführung von Schönheitsbehandlungen für das Gesicht von Frauen nach Anspruch 1, die weiter eine Kühlvorrichtung (43) umfasst, die mit dem Luftkissen zur Versorgung des Kissens (2) mit Kaltluft verbunden ist.

## Revendications

1. Appareil pour administrer des soins de beauté du visage aux femmes comprenant : un masque (1) doublé d'une poche à air (2) à base de polymère perméable à l'eau, qui est muni d'électrodes plates (3) en un matériau souple fixées aux zones du masque (1) à appliquer sur les joues opposées ; une source pulsée basse fréquence (5) reliée aux électrodes plates (3) du masque (1) ; et un compresseur (4) et un humidificateur (45), tous deux reliés à la poche à air (2) pour alimenter la poche à air (2) en vapeur pour le gonflement et le dégonflement.

2. Appareil pour administrer des soins de beauté du visage aux femmes selon la revendication 1 dans laquelle ledit appareil comprend, en outre, un vibrateur (11) fixé au masque (1) pour la vibration.

3. Appareil pour administrer des soins de beauté du visage aux femmes selon la revendication 1 dans laquelle ledit appareil comprend, en outre, un réchauffeur (44) relié à la poche à air (2) pour alimenter la poche à air (2) en air tiède.

4. Appareil pour administrer des soins de beauté du visage aux femmes selon la revendication 1 dans laquelle ledit appareil comprend, en outre, un refroidisseur (43) relié à la poche à air (2) pour alimenter la poche à air (2) en air froid.
